# EUROPÄISCHE PATENTSCHRIFT

(11) **EP 2 229 202 B1**
(45) Veröffentlichungstag und Bekanntmachung des Hinweises auf die Patenterteilung: **06.11.2013**
(21) Anmeldenummer: 08868562.3
(22) Anmeldetag: 04.12.2008
(51) Int. Cl.: A61M 5/178

(54) **EINWEGINJEKTOR MIT ZWEIKOLBIGEM ZWEIKAMMERSYSTEM**
DISPOSABLE INJECTOR WITH A DUAL-PISTON TWO-CHAMBER SYSTEM
INSTRUMENT D'INJECTION À USAGE UNIQUE AVEC SYSTÈME À DEUX CHAMBRES ET DEUX PISTONS

(30) Priorität: 01.01.2008 DE 102008003103
(43) Veröffentlichungstag der Anmeldung: 22.09.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/010250
(87) Internationale Veröffentlichungsnummer: WO 2009/083089

(56) Entgegenhaltungen:
- EP-A- 0 692 235
- DE-A1- 1 913 926
- US-A1- 2003 055 376

## Beschreibung

Die Erfindung betrifft einen Einweginjektor mit einem Gehäuse, einer daran angeordneten - zumindest zeitweise befüllbaren - injektorseitigen, ersten Zylinder-Kolben-Einheit und einem dieser vorgelagerten lösbaren Behälteradapter, wobei der Behälteradapter eine ebenfalls zumindest zeitweise befüllbare zweite Zylinder-Kolben-Einheit trägt.

Aus der WO 96/19252 ist ein für einen Injektor konzipiertes System mit zwei Zylinder-Kolben-Einheiten bekannt. Die injektorseitige, erste Zylinder-Kolben-Einheit lagert den Wirkstoff, während die vorgelagerte, zweite Zylinder-Kolben-Einheit mit einem Lösemittel befüllt ist. Zum Herstellen der Lösung wird das Lösemittel in die injektorseitige Zylinder-Kolben-Einheit gepumpt. Anschließend wird der Behälteradapter vom Injektor getrennt.

Aus der DE 19 13 926 A1 ist eine Zweikammerspritze, die aus einem miteinander verbundenen Spritzenkörper und einem Substanzbehälter besteht, bekannt. Dabei befindet sich die Kanüle außerhalb und zwischen diesen Behältern. Der Spritzenkörper, der das Lösungsmittelbehältnis aufnimmt ist an seiner Vorderseite durch eine Membran abgeschlossen. Der Spritzenkörper besitzt in seiner Bohrung eine Nadelhalterung, die eine durchgehende Kanüle trägt und läuft an ihrer dem Trockenzubstanzbehälter zugewandten Seite konisch aus, wobei das aufgeschraubte Verbindungsstück an seiner Vorderseite innen durch eine Membran verschlossen ist.

Der vorliegenden Erfindung liegt die Problemstellung zugrunde, einen modular aufgebauten Einweginjektor zu entwickeln, der eine Flüssigkeit und einen Wirkstoff jeweils separat steril lagert und einen Raum zur Verfügung stellt, in dem der Wirkstoff für die Applikation in der Flüssigkeit gelöst oder mit der Flüssigkeit gemischt wird.

Diese Problemstellung wird mit den Merkmalen der Ansprüche 1, 10 und 11 gelöst.

Der Kolben der ersten Zylinder-Kolben-Einheit ist in einem rückseitig - im Auslieferungszustand - verschlossenen Zylinder sog-und druckgesteuert bewegbar angeordnet. Der Behälteradapter ist über ein Kuppelelement, in dem er längsverschiebbar angeordnet ist, lösbar am Einweginjektor befestigt. Der Behälteradapter hat einen Adapterbereich und einen Behälterbereich, wobei beide Bereiche durch einen Zwischenboden mit Durchgangsbohrung getrennt sind. Zwischen dem Zwischenboden und dem injektorseitigen Zylinder sitzt ein dicht im Bereich der vorderen Stirnfläche des injektorseitigen Zylinders anliegender, eine Injektionsnadel aufweisender, Nadelträger. Im Behälteradapter ist rückseitig die zweite Zylinder-Kolben-Einheit angeordnet, die vorderseitig - im Auslieferungszustand - mit einem elastischen Stopfen und rückseitig mit einem, gegen Einfahren lösbar gesicherten Kolben dicht verschlossen ist. Die Spitze der Injektionsnadel endet abgedichtet im Stopfen. Der Behälteradapter ist - zur Herstellung einer hydraulischen Verbindung zwischen dem Innenraum des injektorseitigen Zylinders und dem Innenraum des anderen Zylinders - gegen den Nadelträger im Kuppelelement verschiebbar gelagert.

Bei dem Verfahren zur Herstellung einer Lösung aus einem flüssigen Lösemittel und einem Wirkstoff in und an einem Einweginjektor, ist vor der Herstellung der Lösung das Lösemittel in einer injektorseitigen, ersten Zylinder-Kolben-Einheit gelagert, während der Wirkstoff in einer unter Vakuum stehenden, der Zylinder-Kolben-Einheit vorgelagerten, zweiten Zylinder-Kolben-Einheit enthalten ist. Vor der ersten Zylinder-Kolben-Einheit ist temporär eine Injektionsnadel angeordnet, die an der zweiten Zylinder-Kolben-Einheit abgedichtet ist. Die zweite Zylinder-Kolben-Einheit wird - zur Herstellung einer Verbindung zwischen dem Innenraum des injektorseitigen Zylinders und dem Innenraum des anderen Zylinders - unter einem Aufstechen gegen die erste Zylinder-Kolben-Einheit verlagert. Das Lösemittel strömt in den Innenraum der zweiten Zylinder-Kolben-Einheit über und löst dort den Wirkstoff unter Bildung der Lösung. Die Lösung wird in die erste Zylinder-Kolben-Einheit mit dem Kolben der zweiten Zylinder-Kolben-Einheit gepumpt.

Bei einem alternativen Verfahren ist der Kolben der zweiten Zylinder-Kolben-Einheit zu mindestens 80% in den Zylinder eingeschoben. In dem verbleibenden Zylinderrestraum, in dem kein Vakuum herrscht, befindet sich der z.B. gefriergetrocknete Arzneistoff. Um nun während des Verfahrens das Lösungsmittel aus der ersten Zylinder-Kolben-Einheit in die zweite überströmen zu lassen, wird der Kolben der zweiten Zylinder-Kolben-Einheit zur Unterdruckerzeugung zurückgezogen. Ggf. kann er in seiner Endlage - einen Restunterdruck haltend - verrastet werden. Zum Fördern der Lösung in den Innenraum der ersten Zylinder-Kolben-Einheit wird durch ein einschiebendes Betätigen des Kolbens die Verrastung zuvor gelöst.

Mit der Erfindung wird hier beispielsweise ein nadelfreier Einmalinjektor vorgestellt, der zwei Zylinder-Kolben-Einheiten umfasst. Eine erste Zylinder-Kolben-Einheit ist im Injektor integriert und eine zweite ist räumlich vor der injektorseitigen Spritzdüse abnehmbar angeordnet. In der ersten Zylinder-Kolben-Einheit wird ein Lösemittel, z.B. Wasser für Infusionszwecke, steril gelagert. In der zweiten befindet sich z.B. ein ebenfalls steril verpackter, gefriergetrockneter Arzneistoff. Unmittelbar vor der Benutzung des Einweginjektors wird das Wasser in die zweite Zylinder-Kolben-Einheit zum Arzneistoff gefördert. Dort bildet sich eine Lösung, eine Suspension oder eine Emulsion.

Diese Flüssigkeit wird in die erste Zylinder-Kolben-Einheit umgepumpt, um dann injiziert werden zu können. Beim Umpumpen gelangen keine Verklumpungen in den Zylinderrraum der ersten Zylinder-Kolben-Einheit, wodurch ein präziser Injektionsstrahl garantiert wird.

Die zweite Zylinder-Kolben-Einheit ist der ersten hydraulisch vorgelagert. Gemäß des Ausführungsbeispiels sitzt sie räumlich vor der ersten Zylinder-Kolben-Einheit. Die zweite Zylinder-Kolben-Einheit kann jedoch auch seitlich am Injektor angeordnet werden. In diesem Fall würden z.B. beide Zylinder-Kolben-Einheiten parallel nebeneinander sitzen.

Weitere Einzelheiten der Erfindung ergeben sich aus den Unteransprüchen und der nachfolgenden Beschreibung eines schematisch dargestellten Ausführungsbeispiels.
- Figur 1:: Einweginjektor mit zwei Druckstäben und konischer Bundfläche;
- Figur 2:: Ausschnittvergrößerung zu Figur 9;
- Figur 3:: Ausschnittvergrößerung zu Figur 9, jedoch um 90 Winkelgrade versetzt;
- Figur 4:: Ausschnittvergrößerung zu Figur 1, der injektorseitige Zylinderinnenraum lagert Lösemittel, der externe ein Lyophilisat;
- Figur 5:: wie Figur 4, jedoch befinden sich im externen Zylinderinnenraum die Lösung aus Lösemittel und Lyophilisat;
- Figur 6:: wie Figur 4, jedoch befindet sich die Lösung im injektorseitigen Zylinderinnenraum;
- Figur 7:: wie Figur 1, jedoch entsichert und betätigt;
- Figur 8:: wie Figur 2, jedoch nach dem Medikamentenausstoß.

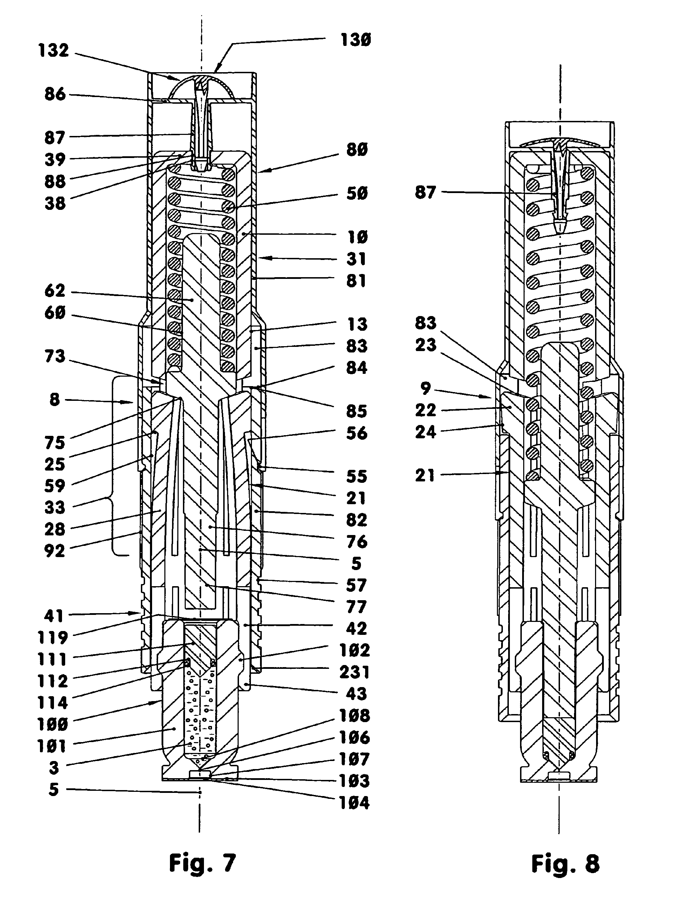

Die Figuren 7 und 8 zeigen einen Einweginjektor mit einem Gehäuse (10), in dem oder an dem - jeweils zumindest bereichsweise - mindestens ein mechanischer Federenergiespeicher, mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist, wobei der Federenergiespeicher (50) mindestens ein vorgespanntes Federelement umfasst, wobei zumindest ein Teil des Kolbenbetätigungsstempels (60) zwischen dem Federenergiespeicher (50) und dem Kolben (111) der Zylinder-Kolben-Einheit (100) positioniert ist.

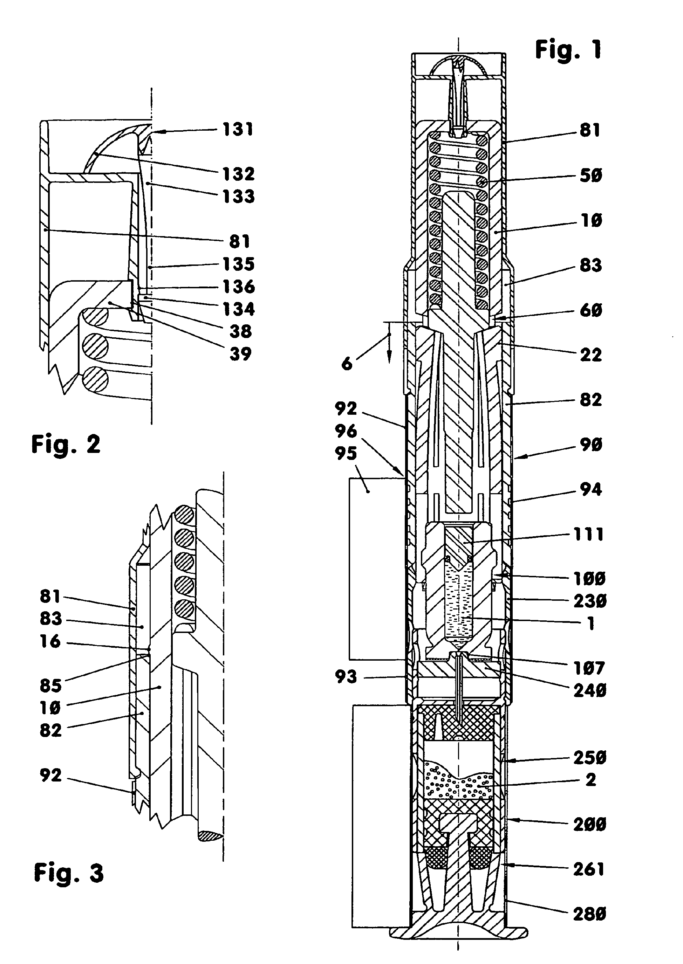

Nach den Figuren 1 und 4 bis 6 ist räumlich vor dem Gehäuse (10) und vor der Zylinder-Kolben-Einheit (100) ein demontierbarer Behälteradapter (200) angeordnet, der ebenfalls eine zumindest zeitweise befüllbare Zylinder-Kolben-Einheit (250) lagert.

Das einteilige Gehäuse (10) des nadelfreien Injektors ist ein topfförmiger, unten offener Hohlkörper mit obenliegendem Boden (39), vgl. Figur 7. Es wird z.B. aus einem glasfaserverstärkten Polyamid durch Spritzgießen gefertigt. Das Gehäuse (10) ist in zwei Funktionsbereiche aufgeteilt, das ist zum einen der obere Mantelbereich (31) und zum anderen der untere Fixierbereich (41). Im Mantelbereich (31) hat das Gehäuse (10) z.B. zwei einander gegenüberliegende fensterartige Durchbrüche (33), vgl. Figur 1. Am unteren Rand des einzelnen Durchbruches (33) ist jeweils ein Druckstab (21) gelenkig gelagert.

Die Druckstäbe (21) sind am Gehäuse (10) angeformt und federn als elastische Biegebalken (28) nach außen. Die Anformstelle für die Druckstäbe (21) liegt knapp oberhalb des Fixierbereichs (41). Zur Ausbildung des jeweiligen Druckstabs (21) befindet sich im Mantelabschnitt (31) ein schmaler, zumindest annähernd u-förmiger Spalt, der den einzelnen Druckstab seitlich und oben umgibt. Der Druckstab (21) hat auf ca. 80% seiner Länge die Wandstärke und die Krümmung der Wandung des Gehäuses (10). Speziell dieser Bereich hat u.a. auch die Funktion des federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 8 ist der Druckstab (21) im unverformten Zustand dargestellt.

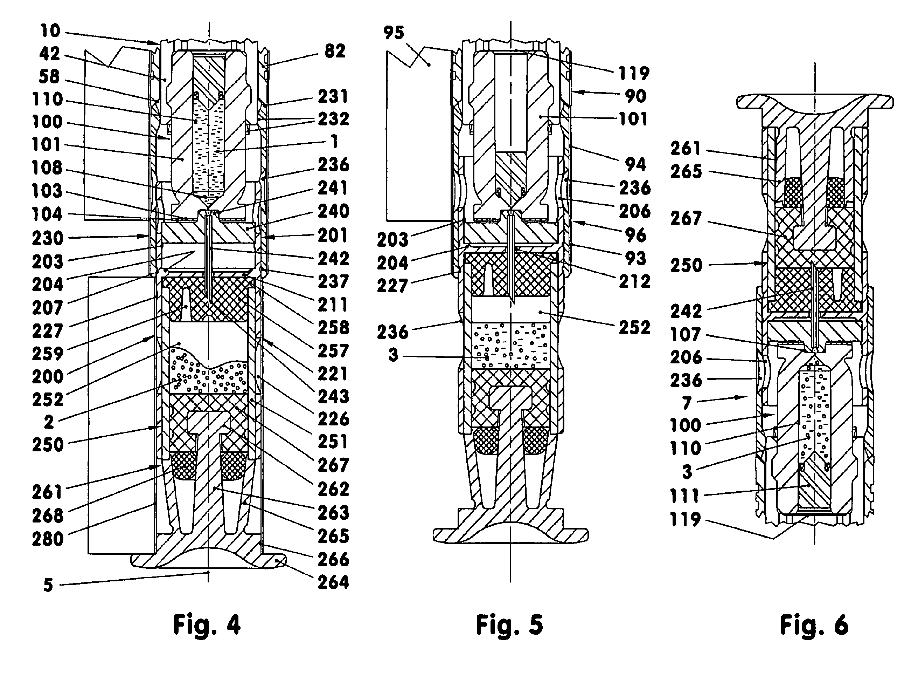

Das hier obere freie Ende des einzelnen Druckstabs (21) wird durch den radial nach außen abstehenden Nocken (22) gebildet. Letzterer hat zumindest eine Abstützfläche (23) und eine radiale Anlagefläche (24). Nach Figur 5 liegt auf der Abstützfläche (23) der Stempelteller (73) des gespannten Einweg-Injektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Druckstäbe (21) oder die Bundfläche (75) zumindest im Kontaktbereich eine keramische Panzerung oder sind z.B. durch eine aufgeklebte kegelstumpfmantelförmige Unterlegscheibe verstärkt.

Die Anlagefläche (24) der Nocken (22) des unverformten Biegebalkens (28) ist Teil eines Kegelstumpfmantels, dessen maximaler Durchmesser z.B. 3 bis 4 Millimeter größer als der Außendurchmesser des Gehäuses (10) ist, vgl. Figur 8. Die Anlagefläche (24) kontaktiert bei gespanntem Einweginjektor die Innenwandung (59) des hülsenartigen Auslöseelements (82). Ggf. hat - zur Minimierung der Flächenpressung - die Anlagefläche (24) eine Krümmung, die der Innenwandung (59) entspricht.

Unterhalb des Mantelabschnitts (31) befindet sich der Fixierbereich (41) zur Aufnahme der einbaubaren Zylinder-Kolben-Einheit (100). Der Fixierbereich (41) umfasst z.B. acht parallel zur Mittellinie (5) ausgerichtete Federhaken (42). Die Federhaken (42) haben jeweils einen mindestens zweiflankigen Hintergriff (43) zur spielfreien Aufnahme der Zylinder-Kolben-Einheit (100). Die einander gegenüber liegenden Flanken des Hintergriffs (43) schließen einen Winkel von z.B. 90 Winkelgraden ein. Die Länge und die Federrate der Federhaken (42) ist so dimensioniert, dass der Zylinder (101) ohne plastische Verformung der Federhaken (42) eingebaut werden kann.

Die Zylinder-Kolben-Einheit (100) besteht im Ausführungsbeispiel aus einem, mit einem flüssigen Lösemittel (1) befüllten Zylinder (101), vgl. Figur 1, in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Der Zylinder (101) ist z.B. ein klarsichtiger, dickwandiger Topf, dessen ggf. zylindrische Außenwandung eine beispielsweise umlaufende Rastrippe (102) trägt, vgl. Figur 7, die an den Flanken des Hintergriffs (43) der Federhaken (42) formsteif anliegt. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101). Diese Stirnfläche (103) kann zur Erhöhung der Applikationssicherheit zusätzlich mit einem Klebering (104) versehen werden.

Der Zylinder (101) ist rückseitig mit einer Sterilfiltermembrane (119) steril verschlossen.

Zwischen dem Stempelteller (73) und dem oben liegenden Boden (39) des Gehäuses (10) sitzt vorgespannt die Schraubendruckfeder (50). Die Federkraft wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50). Unterhalb des Stempeltellers (73) befindet sich zentral in der Verlängerung des Führungszapfens (62) der Kolbenschieber (76), der bei einer Betätigung des Einmal-Injektors auf den Kolben (111) wirkt. Im Ausführungsbeispiel endet der Kolbenschieber (76) z.B. 2 bis 4 Millimeter oberhalb der Sterilfiltermembrane (119) der Zylinder-Kolben-Einheit (100).

Der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Die Figuren stellen einen Druckstab-Injektor mit einer das Gehäuse (10) fast vollständig umschließenden Auslöseeinheit (80) dar. An dem Auslöseelement (82) ist dazu eine Auslösekappe (81) befestigt, die das hintere Ende des Gehäuses (10) umgibt, vgl. auch Figuren 1, 7 und 8.

Das teilweise das Gehäuse (10) und die Zylinder-Kolben-Einheit (100) umgebende Auslöseelement (82) ist eine Auslösehülse. Die im Wesentlichen zylindrische, z.B. aus ABS gefertigte, Auslösehülse (82) hat an ihrem hinteren Ende als Stirnfläche eine Rücksprungflanke (84), die hier eine zur Mittellinie (5) des Injektors normale Ebene darstellt. Der Übergang zwischen der beispielsweise zylindrischen Innenwandung des Auslöseelements (82) und der Rücksprungflanke (84) ist z.B. als scharfkantige Kante (85) ausgebildet.

Im unteren Bereich des Auslöseelements (82) befinden sich in dessen Außenwandung mehrere umlaufende Rillen (57) oder eine andere vergleichbare Struktur. Die Rillen (57) haben gegeneinander z.B. gleiche Abstände und erstrecken sich über 10 bis 30 Millimetern Länge des Auslöseelements (82).

Die Auslösekappe (81) ist über das hintere Ende des Auslöseelements (82) geschoben. Unmittelbar oberhalb der Rücksprungflanke (84) des Auslöseelements (82) befindet sich eine Aufweitung (83). Diese Aufweitung (83) ist im Bezug auf das Gehäuse (10) genau so positioniert und dimensioniert, dass sie die beim Auslösevorgang zurückweichenden, nach außen gedrängten Druckstäbe (21) mit ihren Nocken (22) aufnehmen kann, vgl. Figur 8. Die Innenkontur der Aufweitung (83) ist z.B. ein umlaufender Kanal.

Anstelle dieser Aufweitung (83) können bei einem nichtrotations-symmetrischen Auslöseelement (82) pro Druckstab (21) auch partielle Aufweitungen oder nicht abgedeckte Öffnungen vorhanden sein.

Nach Figur 7 liegen die Nocken (22) mit ihren außen liegenden Anlageflächen (24) an der Innenwandung (59) des Auslöseelements (82) sichernd an. Oberhalb der Aufweitung (83) liegt die Auslösekappe (81) gleitfähig an der Außenwandung (13) des Gehäuses (10) an.

Zur Befestigung der Auslösekappe (81) am Auslöseelement (82) hat das Auslöseelement (82) beispielsweise eine Ringnut (56), in die ein Umlaufsteg oder Rastnocken (55) der Auslösekappe (81) eingreifen. Die Auslösekappe (81) kann - zur Erleichterung der Montage - z.B. zweifach bereichsweise längsgeschlitzt sein, vgl. Figuren 7 und 8.

Am hinteren Ende hat die Auslösekappe (81) einen vertieft sitzenden Kappenboden (86). Am Kappenboden (86) sind um eine zentrische Bohrung herum z.B. mehrere nach innen ragende Rastzungen (87) angeformt. Die Rastzungen (87) weisen an ihren unteren Enden jeweils Zungenkerben (88) auf, die den Rand einer zentralen Bohrung (38) des Gehäusebodens (39) umgreifen.

Die Rastzungen (87) werden durch einen Sperrer (131) einer Druckknopfsicherung (130), vgl. Figur 2, in der den Boden (39) bereichsweise umgreifenden Position fixiert, so dass sich die Auslösekappe (81) in Kombination mit dem Auslöseelement (82) nicht gegenüber dem Gehäuse (10) in Längsrichtung bewegen kann.

Der Sperrer (131) hat einen elastischen, teilkugelschalenförmigen Sperrerknopf (132), an dem ein Sperrerbolzen (133) angeformt ist. Letzterer trägt an seinem unteren, freien Ende einen Blockierbund (134), der sich gegen eine Taille (135) absetzt. Der Blockierbund (134) hält die Rastzungen (87) in ihrer sperrenden Lage, vgl. Figur 2, und rastet hinter einem Raststeg (136) sicher ein.

Wird der Sperrer (131) durch Niederdrücken betätigt, springen die federelastischen Rastzungen (87) hinter den Blockierbund (134) und legen sich an der Taille (135) an. Der Sperrer (131) verharrt dauerhaft in seiner betätigten Lage, vgl. Figur 8. Die neue Hüllfläche der Rastzungen (87) hat nun einen Außendurchmesser, der kleiner ist als der Innendurchmesser der Bohrung (38). Folglich ist die mechanische Kopplung zwischen dem Auslöseelement (82) und dem Gehäuse (10) gelöst.

Um das Gehäuse (10) zusammen mit dem Federelement (50) und dem Kolbenbetätigungsstempel (60) bei der Montage im Auslöseelement (82) verliersicher fixieren zu können, hat das Gehäuse (10) in einem Bereich zwischen den Nocken (22) eine linsenförmige Erhebung (16), vgl. Figur 3, über die das Gehäuse (10) an der Kante (85) des Auslöseelements (82) anliegt.

Bei dem hier gezeigten Gehäuse (10) haben die Stützstäbe (21) Nocken (22) mit besonderen Hintergriffsflanken (25). Diese Hintergriffsflanken (25) liegen bei verformten Stützstäben (21) zumindest annähernd in einer zur Mittellinie (5) normalen Ebene. Demnach rasten sie beim Auslösen des Injektors schlagartig über die Kante (85). Nach dem Auslösen liegen sie zudem fest verrastet an der Rücksprungflanke (85) des Auslöseelements (82) an.

An der unteren z.B. kegelstumpfmantelförmigen Stirnfläche (58), vgl. Figur 4, des Auslöseelements (82) liegt das am Zylinder (101) der Zylinder-Kolben-Einheit (100) zentrierte Kuppelelement (230) an. Die zumindest bereichsweise annähernd zylindrische Außenfläche des Kuppelelements (230) hat den gleichen Außendurchmesser wie die ebenfalls zylindrische Außenfläche des Auslöseelements (82) in der Nähe ihrer Stirnfläche (58).

Das z.B. zylinderrohrförmige Kuppelelement (230) umhüllt nicht nur bereichsweise den Zylinder (101) und liegt dabei an dem Auslöseelement (82) an, sondern stützt sich zusätzlich am Gehäuse (10) ab. Dazu hat es in der Nähe der Stirnfläche (231) mehrere an der Innenwandung angeordnete Anlagestege (232). Die radial nach innen ragenden Anlagestege (232) sind parallel zur Mittellinie (5) ausgerichtet und kontaktieren die Federhaken (42) des Gehäuses (10).

Das Kuppelelement (230) weist zwei einander gegenüberliegende Fenster (236) auf. Die Fenster (236) haben eine Breite, die mindestens dem Durchmesser des Kolbens (111) entspricht. Die Fenstermitten befinden sich in der Höhe des Zylinderbodens (108), vgl. auch Figur 6. Die Innenwandung des Kuppelelements (230) ist im Wesentlichen zylindrisch und glattwandig. Sie bildet die Führungsfläche für den Behälteradapter (200). Am unteren Rand hat sie z.B. mehrere am Umfang verteilte Rastnoppen (237). In der Führungsfläche befindet sich ggf. ein hier nicht dargestellter - zur Mittellinie (5) paralleler - Längssteg, der zur Verdrehsicherung in eine entsprechende Nut des Behälteradapters (200) eingreift.

Der Behälteradapter (200) ist ein büchsenartiges Bauteil, das eine Zylinder-Kolben-Einheit (250) in einem Behälterbereich (221) aufnimmt. Zugleich hat er einen hülsenförmigen Adapterbereich (201) mit dem er längsverschiebbar im Kuppelelement (230) sitzt. Innen führt der Adapterbereich (201) längsverschiebbar einen Nadelträger (240).

Der Adapterbereich (201) ist ein zylindrischer Becher, der zumindest nach Figur 4 das untere Fünftel des Zylinders (101) mit Abstand umgibt. Er hat zwei einander gegenüberliegende z.B. kreisrunde Fenster (206), vgl. Figur 5, mehrere, z.B. vier am Umfang verteilte Anschlagnoppen (203) als obere Anschläge für den Nadelhalter (240) und einen ringförmigen Absatz (204) auf dem zentral aufgebohrten Zwischenboden (211). Die Fenster (206) und der zuvor erwähnte Längssteg des Kuppelelements (230) können entfallen, wenn das Behälteradaptermaterial transparent ist. Der Längssteg kann ferner entfallen, wenn die Fenster (206) zum einen eine rechteckige Form haben und zum anderen mehrere nur durch schmale Stege voneinander getrennte Fenster (206) am Umfang verteilt sind.

An seiner Außenwandung hat der Adapterbereich (201) auf der Höhe des Zwischenbodens (211) eine umlaufende Kerbe (207), in die nach Figur 4 die Rastnoppen (237) eingreifen.

Vor der Stirnseite (103) des Zylinders (101) ist direkt vor der Klebefolie (104) der Nadelträger (240) angeordnet. Letzterer hat einen Zapfen (241), mit dem er abdichtend in der Zylinderausnehmung (107) sitzt. Die untere Außenkante des Nadelträgers (240) liegt auf den Anschlagnoppen (203) auf.

Im zylinderscheibenförmigen Nadelträger (240) steckt zentral, z.B. umspritzt oder eingeklebt, eine kurze Injektionsnadel (242). Nach Figur 4 ragt die Injektionsnadel (242) durch die Bohrung (212) des z.B. ebenen Zwischenbodens (211) hindurch und endet mit ihrer Nadelspitze (243) z.B. mittig in einem unterhalb des Zwischenbodens (211) gelegenen Stopfen (257).

Im rohrförmigen Behälterbereich (221) befinden sich ebenfalls zwei oder vier einander gegenüber liegende Fenster (226). Zudem hat die Außenwandung kurz vor dem Zwischenboden (211) eine umlaufende Kerbe (227), in der nach Figur 5 die Rastnoppen (237) des Kuppelelementes (230) verrasten. Ggf. hat der Behälterbereich (221) am unteren Ende seiner Außenwandung zwei Griffelemente, z.B. in Form einer nach DIN 13098 Teil 2 genormten Zylindergriffplatte.

Im Behälterbereich (221) ist die Zylinder-Kolben-Einheit (250) angeordnet. Ihr Außendurchmesser ist nur geringfügig kleiner als der Innendurchmesser des Behälterbereichs (221). Die Zylinder-Kolben-Einheit (250) ist z.B. im Behälterbereich (221) eingeklebt.

Die Zylinder-Kolben-Einheit (250) hat einen Zylinder, der aus einem transparenten Rohr (251), z.B. einem Glas- oder Kunststoffrohr (COC), und einem elastischen Stopfen (257) gebildet wird. Der Stopfen (257), in Figur 4 liegt er oben, hat einen Flanschrand (258), über den er axial am Glasrohr (251) anliegt. Zum Zylinderinnenraum (252) hin weist der Stopfen (257) eine asymmetrische Einkerbung (259) auf, die ggf. für die Gefriertrocknung der in der Zylinder-Kolben-Einheit (250) aufgenommenen Lösung des Arzneistoffes (2), z.B. ein Protein, notwendig ist.

Nach Figur 4 ist das Glasrohr (251) rückseitig mit einem beweglichen Kolben (261) verschlossen. Der Kolben (261) besteht aus einer Kolbenstange (262), einer hinteren Kolbendruckplatte (264), einem vorderen Stopfenträger (263) und einem darüber gestülpten elastischen Kolbenstopfen (267). Um den Kolben (261) bei einem vakuumisierten Zylinderinnenraum (252) in seiner hinteren Position zu halten, hat der Kolben (261) zusätzlich zwei oder mehrere Rastelemente (265), die z.B. an der Kolbendruckplatte (264) angeformt sind und sich - elastisch nach außen federnd - auf dem hinteren Rand des Glasrohres (251) abstützen. An der Rückseite des Kolbenstopfens (267) sitzt ein elastischer Gummiring (268), der die Rastelemente (265) nach außen drückt.

Die Kolbendruckplatte (264) hat zum Glasrohr (251) hin einen zylindrischen Bund (266), der den gleichen Außendurchmesser hat wie der Behälterbereich (221).

Nach Figur 4 ist der Behälterbereich (221) und der Kolben (261) zwischen dem Kuppelelement (230) und der Kolbendruckplatte (264) mit einer Abwickelbanderole (280) überklebt. Die Abwickelbanderole (280) überdeckt hierbei schützend die Fenster (226) und die Rastelemente (265) des Kolbens (261). Zusätzlich verhindert die Abwickelfolie (280) ein unbeabsichtigtes Einschieben des Behälteradapters (200) in das Kuppelelement (230).

Zur Sicherung des Kuppelelements (230) ist dieses, vgl. Figur 1, über die Banderole (90) mit dem Auslöseelement (82) des Injektors verbunden. Die Banderole (90), ein als Klebeetikett ausgebildeter Originalitätsverschluss, deckt nahezu die gesamte zylindrische Außenwandung des Kuppelelements (230) und die Rillen (57) des Auslöseelements (82) ab.

Die Banderole (90) selbst ist z.B. ein mit einem Klebstoff bereichsweise einseitig beschichteter Papier- und/oder Folienstreifen. Die Banderole (90) besteht aus drei separaten Streifen, die jeweils über eine Perforation (96) oder über eine andere Sollbruchstelle gegeneinander abtrennbar sind. Die jeweils umlaufenden Perforationen (96) liegen oberhalb der Rillen (57) und unterhalb der Fenster (206).

Der obere Streifen ist ein hinteres Randteil (92), der mittlere Streifen ist eine Abreißbanderole (94) mit einer zwei bis drei Zentimeter langen Abreißfahne (95) und der untere Streifen ist ein vorderes Randteil (93). Werden die Abreißfahne (95) und die Abreißbanderole (94) ringsherum vom Auslöseelement (82) und dem Kuppelelement (230) entgegen der Klebehaftung - unter Auftrennung der Perforationen (96) - abgewickelt, hält das Kuppelelement (230) nur noch über Klemmkräfte am Injektor.

Um den Einweginjektor benutzen zu können, muss der in der Zylinder-Kolben-Einheit (250) gelagerte Wirkstoff (2), z.B. ein Lyophilisat, in der im Zylinder (101) der Zylinder-Kolben-Einheit (100) vorhandenen Flüssigkeit (1), z.B. Wasser für Injektionszwecke, bzw. Physiologische Kochsalzlösung, gelöst werden.

Dazu soll die Flüssigkeit (1) in den Behälter (250) gepumpt werden.

In einem ersten Schritt wird die Abwickelbanderole (280) vom Behälterbereich (221) entfernt und der Behälteradapter (200) in das Kuppelelement (230) hineingeschoben, vgl. Figur 5. Hierbei drückt sich die Injektionsnadel (242) durch den Stopfen (257) hindurch, so dass die Nadelspitze (243) im Zylinderinnenraum (252) endet. Die Schubbewegung des Behälteradapters (200) ist beendet, wenn der Absatz (204) am Nadelhalter (240) anliegt. Die Fenster (206) des Behälteradapters (200) überdecken sich mit den Fenstern (236) des Kuppelelements (230). Die Rastnoppen (237) rasten in der umlaufenden Kerbe (227) ein.

Durch das Eindringen der Injektionsnadel (242) in den Zylinderinnenraum (252) kommuniziert dieser über die Injektionsnadel (242) mit dem Zylinderinnenraum (110). Das Vakuum des Zylinderinnenraums (252) saugt die Flüssigkeit aus dem Zylinder (101) der Zylinder-Kolben-Einheit (100). Da die rückseitige Abdeckung des Zylinders (101) eine Sterilfiltermembrane (119) ist, kann der angesaugte Kolben (111) der Flüssigkeit (1) folgen und kommt am Zylinderboden (108) zur Anlage. Im Innenraum (252) löst sich das Lyophilisat (2) in der Flüssigkeit (1). Der Lösevorgang kann über die Fenster (226) beobachtet werden.

In einem zweiten Schritt wird, sobald das Lyophilisat (2) gelöst ist, die Abreißbanderole (94) entfernt. Die Rillen (57) des Auslöseelements (82) werden damit sichtbar. Nun wird der Injektor so positioniert, dass die Zylinder-Kolben-Einheit (100) unterhalb der Zylinder-Kolben-Einheit (250) liegt, vgl. Figur 6. Danach soll die neu entstandene Lösung (3) durch die Injektionsnadel (242) hindurch in den Zylinderinnenraum (110) gepumpt werden. Dazu wird der Kolben (261) durch ein radiales Eindrücken der Rastelemente (265) zunächst entsichert. Aufgrund des Restvakuums legt sich der Kolbenstopfen (267) auf die Oberfläche der Lösung (3). Nun wird durch leichten Druck auf den Kolben (261) die Lösung (3) in den Zylinderinnenraum (110) umgepumpt. Die Lösung (3) schiebt den Kolben (111) vor sich her. Ein blasenfreies Befüllen des Zylinderinnenraumes (110) wird über die Fenster (206, 236) im Durchlicht geprüft. In der Regel wird ein kleiner Teil der Lösung (3) in das Glasrohr (251) zurückgesaugt, so dass zudem der Kolben (111) nicht an der Sterilfiltermembrane (119) anliegt.

In einem dritten Schritt wird das Kuppelelement (230) zusammen mit dem Behälteradapter (200) - bezogen auf Figur 6 - nach oben vom Gehäuse (10) abgezogen. Der Injektor bleibt trotzdem gesichert.

Nach dem Aufsetzen des Injektors auf die Injektionsstelle muss in einem letzten Schritt, z.B. mit dem Daumen der den Injektor haltenden Hand, der Sperrerknopf (132) gedrückt werden, um das Auslöseelement (82) zusammen mit der Auslösekappe (81) bewegen zu können. Nun kann das Auslöseelement (82) in Richtung der Zylinder-Kolben-Einheit (100) verschoben werden. Bei diesem Vorgang gleitet das Auslöseelement (82) auf der Außenwandung (13) des Gehäuses (10) linear nach unten, also in Richtung der Injektionsstelle. Die Anlageflächen (24) der Druckstäbe (21) rutschen über die Kante (85) und springen unter der Kraft des Federelements (50) entsichernd radial nach außen in die Aufweitung (83). Die Druckstäbe (21) haben sich elastisch nach außen gebogen und befinden sich nun in ihrer eigentlichen Ausgangslage. Die nun nicht mehr verformten Druckstäbe (21) geben den Kolbenbetätigungsstempel (60) frei, so dass sich der Kolbenschieber (76) unter der Wirkung des Federelements (50) ruckartig auf die Sterilfiltermembrane (119) des Zylinders (101) zubewegt. Die Sterilfiltermembrane (119) wird durchschlagen und der Kolben (111) zum Entleeren des Zylinders (101) nach unten bewegt, vgl. Figur 8. Der Zylinder (100) wird entleert.

Anstelle einer linearen Gleitbewegung des Auslöseelements (82) auf dem Gehäuse (10) kann auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Bei den in den Figuren dargestellten Varianten ist die einzelne Kontaktzone zwischen dem Druckstab (21) und dem Stempelteller (73), als Flächen (23) und (75) ausgeführt, die gleitfähig einander kontaktieren. In einer besonderen Ausgestaltung kann in jeder Fläche (23) der einzelnen Druckstäbe (21) eine Walze gelagert werden, die bei einer Betätigung des Injektors an der Fläche (75) des Stempeltellers wälzgelagert, also reibungsarm, abrollt.

Mit Ausnahme des Federelements (50), einer ggf. vorhandenen Kolbenplatte und der beispielsweise vorhandenen Lagerwalzen der Stützstäbe (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Wasser für Infusionszwecke, Lösemittel
- 2: Lyophilisat, Wirkstoff, Arzneistoff
- 3: Injektionslösung
- 5: Mittellinie des Injektors, Längsrichtung
- 6: Auslösebewegungsrichtung von (82), Abwärtsbewegung Richtungspfeil
- 7: Umpumpposition
- 8: Sperrstellung
- 9: Lösestellung, Auslösestellung

- 10: Gehäuse, einteilig
- 13: Außenfläche, zylindrisch
- 16: Erhebung, linsenförmig

- 21: Druckstäbe, Stützstäbe
- 22: Nocken
- 23: Abstützfläche
- 24: Anlagefläche
- 25: Hintergriffsflanke
- 28: Biegebalken

- 31: Mantelbereich
- 33: Durchbrüche
- 38: Bohrung
- 39: Boden

- 41: Fixierbereich für die Zylinder-Kolben-Einheit
- 42: Federhaken
- 43: Hintergriff

- 50: Federelement, Schraubendruckfeder, Federenergiespeicher
- 55: Rastnocken
- 56: Ringnut von (82)
- 57: Rillen von (82)
- 58: Stirnfläche von (82)
- 59: Innenwandung von (82)

- 60: Kolbenbetätigungsstempel
- 62: Führungszapfen
- 73: Stempelteller
- 75: Bundfläche, konisch
- 76: Kolbenschieber
- 77: Kolbenschieberstirnfläche, kegelmantelförmig

- 80: Auslöseeinheit
- 81: Auslösekappe
- 82: Auslöseelement
- 83: Aufweitung
- 84: Rücksprungflanke
- 85: Kante, scharfkantig
- 86: Kappenboden
- 87: Rastzungen
- 88: Zungenkerbe

- 90: Originalitätsverschluss, Banderole, Sicherungselement Klebeetikett
- 92: Randteil, hinten; Etikettteil
- 93: Randteil, vorn; Etikettteil
- 94: Abreißbanderole
- 95: Abreißfahne
- 96: Perforationen, Sollbruchstellen

- 100: Zylinder-Kolben-Einheit, erste, injektorseitig
- 101: Zylinder, injektorseitig
- 102: Rastrippe
- 103: Stirnfläche
- 104: Klebering
- 106: Bohrung, Düse
- 107: Ausnehmung in der Stirnfläche
- 108: Zylinderboden
- 110: Zylinderinnenraum
- 111: Kolben
- 112: Ringnut
- 114: Dichtring, Dichtung
- 119: Sterilfiltermembrane

- 130: Druckknopfsicherung, Sicherungselement
- 131: Sperrer
- 132: Sperrerknopf
- 133: Sperrerbolzen
- 134: Blockierbund
- 135: Taille
- 136: Raststeg

- 200: Behälteradapter
- 201: Adapterbereich
- 203: Anschlagnoppen
- 204: Absatz, ringförmig
- 206: Fenster, beidseitig
- 207: Kerbe, umlaufend
- 211: Zwischenboden
- 212: Bohrung, zentral
- 221: Behälterbereich
- 226: Fenster
- 227: Kerbe, umlaufend

- 230: Kuppelelement, rohrförmig
- 231: Stirnfläche
- 232: Anlagestege
- 236: Fenster
- 237: Rastnoppen

- 240: Nadelträger
- 241: Zapfen
- 242: Injektionsnadel
- 243: Nadelspitze

- 250: Zylinder-Kolben-Einheit, zweite
- 251: Rohr, Glasrohr, Kunststoffrohr
- 252: Zylinderinnenraum
- 257: Stopfen, elastisch, Gummistopfen
- 258: Flanschrand
- 259: Einkerbung

- 261: Kolben
- 262: Kolbenstange
- 263: Stopfenträger
- 264: Kolbendruckplatte
- 265: Rastelemente
- 266: Bund
- 267: Kolbenstopfen
- 268: Gummiring, Elastomerfeder

- 280: Abwickelbanderole

## Patentansprüche

1. Einweginjektor mit einem Gehäuse (10), einer daran angeordneten - zumindest zeitweise befüllbaren - Zylinder-Kolben-Einheit (100) und einem dieser vorgelagerten lösbaren Behälteradapter (200), wobei der Behälteradapter (200) eine ebenfalls zumindest zeitweise befüllbare Zylinder-Kolben-Einheit (250) lagert, **dadurch gekennzeichnet,**
- **dass** der Kolben (111) der Zylinder-Kolben-Einheit (100) in einem rückseitig - im Auslieferungszustand - verschlossenen Zylinder (101) sog- und druckgesteuert bewegbar angeordnet ist,
- **dass** der Behälteradapter (200) über ein Kuppelelement (230), in dem er längsverschiebbar sitzt, lösbar am Einweginjektor befestigt ist,
- **dass** der Behälteradapter (200) einen Adapterbereich (201) und einen Behälterbereich (221) hat, wobei beide Bereiche (201, 221) durch einen Zwischenboden (211) mit Durchgangsbohrung (212) getrennt sind,
- **dass** zwischen dem Zwischenboden (211) und dem Zylinder (101) ein dicht im Bereich der vorderen Stirnfläche (103) des Zylinders (101) anliegender, eine Injektionsnadel (242) aufweisender, Nadelträger (240) sitzt,
- **dass** im Behälteradapter (200) rückseitig die zweite Zylinder-Kolben-Einheit (250) angeordnet ist, die vorn - im Auslieferungszustand - mit einem elastischen Stopfen (257) und hinten mit einem, gegen Einfahren lösbar gesicherten, Kolben (261) dicht verschlossen ist,
- **dass** die Spitze (243) der Injektionsnadel (242) im Stopfen (257) abgedichtet endet,
- **dass** der Behälteradapter (200) - zur Herstellung einer Verbindung zwischen dem Innenraum (110) des injektorseitigen Zylinders (101) und dem Innenraum (252) des anderen Zylinders (251, 257) - gegen den Nadelträger (240) im Kuppelelement (230) verschiebbar gelagert ist.

2. Einweginjektor gemäß Anspruch 1, **dadurch gekennzeichnet, dass** der Zylinder (101) der ersten Zylinder-Kolben-Einheit (100) - im Auslieferungszustand - rückseitig mit einer Sterilfiltermembrane (119) gasdurchlässig verschlossen ist.

3. Einweginjektor gemäß Anspruch 1, **dadurch gekennzeichnet, dass** der Nadelträger (240) längsverschiebbar im Adapterbereich (201) geführt ist.

4. Einweginjektor gemäß Anspruch 1, **dadurch gekennzeichnet, dass** der Adapterbereich (201) vorderseitig den injektorseitigen Zylinder (101) zumindest bereichsweise umgibt.

5. Einweginjektor gemäß Anspruch 1, **dadurch gekennzeichnet, dass** die zweite Zylinder-Kolben-Einheit (250) fest im Behälteradapter (200) sitzt.

6. Einweginjektor gemäß Anspruch 1, **dadurch gekennzeichnet, dass** die zweite Zylinder-Kolben-Einheit (250) aus einem, mit einem elastischen Stopfen (257) und einem Kolben (261) verschlossenen, transparenten Glasrohr (251) besteht.

7. Einweginjektor gemäß Anspruch 1, **dadurch gekennzeichnet, dass** der Kolben (261) der zweiten Zylinder-Kolben-Einheit (250) federnde oder federbelastete Rastelemente (265) aufweist, die den Kolben (261) in einer ausgefahrenen Position lösbar fixieren.

8. Einweginjektor gemäß Anspruch 1, **dadurch gekennzeichnet, dass** der Behälteradapter (200) im mittleren Bereich des Glasrohres (251) zwei einander gegenüberliegende Fenster (226) aufweist.

9. Einweginjektor gemäß Anspruch 1, **dadurch gekennzeichnet, dass** zum einen der Behälteradapter (200) im injektornahen Bereich zwei weitere einander gegenüberliegende Fenster (206) hat und zum anderen das Kuppelelement (230) zwei einander gegenüber liegende Fenster (236) aufweist, wobei die Fenster (236) in der Umpumpposition (7) des Injektors vor den Fenstern (206) liegen.

10. Verfahren zur Herstellung einer Lösung (3) aus einem Lösemittel (1) und einem Wirkstoff (2) in und an einem Einweginjektor, **dadurch gekennzeichnet,**
- **dass** vor der Herstellung der Lösung (3) das Lösemittel (1) in einer injektorseitigen, ersten Zylinder-Kolben-Einheit (100) gelagert ist, während der Wirkstoff (2) in einer unter Vakuum stehenden, der Zylinder-Kolben-Einheit (100) vorgelagerten, zweiten Zylinder-Kolben-Einheit (250) enthalten ist,
- **dass** vor der ersten Zylinder-Kolben-Einheit (100) temporär eine Injektionsnadel (242) angeordnet ist, die an der zweiten Zylinder-Kolben-Einheit (250) abgedichtet ist,
- **dass** die zweite Zylinder-Kolben-Einheit (250) - zur Herstellung einer Verbindung zwischen dem Innenraum (110) des Zylinders (101) und dem Innenraum (252) des Zylinders (251, 257) - unter einem Aufstechen gegen die erste Zylinder-Kolben-Einheit (100) verlagert wird,
- **dass** das Lösemittel (1) in den Innenraum (252) der zweiten Zylinder-Kolben-Einheit (250) überströmt und dort sich der Wirkstoff (2) unter Bildung der Lösung (3) im Lösemittel (1) löst und
- **dass** die Lösung (3) in die erste Zylinder-Kolben-Einheit (100) mit dem Kolben (261) der zweiten Zylinder-Kolben-Einheit (250) gepumpt wird.

## Claims

1. Disposable injector with a housing (10), a cylinder-piston unit (100) - at least intermittently fillable - arranged thereon and an upstream detachable container adapter (200), wherein the container adapter (200) stores a likewise at least intermittently fillable cylinder-piston unit (250), **characterized in that**,
- the piston (111) of the cylinder-piston unit (100) is movably arranged in a suction- and pressure-controlled manner in a cylinder (101) sealed at the back in the delivery state,
- the container adapter (200) is attached detachably to the disposable injector via a coupling element (230), in which it sits displaceable longitudinally,
- the container adapter (200) has an adapter region (201) and a container region (221), whereby both regions (201, 221) are separated by an intermediate floor (211) with throughhole (212),
- between the intermediate floor (211) and the cylinder (101) a needle holder (240) sits closely adjacent in the region of the front abutting face (103) of the cylinder (101) and having an injection needle (242),
- in the container adapter (200) the second cylinder-piston unit (250) is arranged at the back and is tightly sealed at the front - in the delivery state - with an elastic stopper (257) and at the back with a piston (261), secured detachably against running in,
- the tip (243) of the injection needle (242) terminates sealed in the stopper (257),
- the container adapter (200) is mounted displaceably against the needle holder (240) in the coupling element (230) to make a connection between the interior space (110) of the injector-side cylinder (101) and the interior space (252) of the other cylinder (251, 257).

2. Disposable injector according to Claim 1, **characterized in that** the cylinder (101) of the first cylinder-piston unit (100) is sealed gas-permeably in the delivery state to the rear with a sterile filter membrane (119).

3. Disposable injector according to Claim 1, **characterized in that** the needle holder (240) is guided longitudinally displaceably in the adapter region (201).

4. Disposable injector according to Claim 1, **characterized in that** on the front side the adapter region (201) encloses the injector-side cylinder (101) at least in certain areas.

5. Disposable injector according to Claim 1, **characterized in that** the second cylinder-piston unit (250) sits firmly in the container adapter (200).

6. Disposable injector according to Claim 1, **characterized in that** the second cylinder-piston unit (250) comprises a transparent glass tube (251) sealed with an elastic stopper (257) and a piston (261).

7. Disposable injector according to Claim 1, **characterized in that** the piston (261) of the second cylinder-piston unit (250) has resilient or springloaded latching elements (265), which detachably fix the piston (261) in an extended position.

8. Disposable injector according to Claim 1, **characterized in that** the container adapter (200) has two opposite windows (226) in the central region of the glass tube (251).

9. Disposable injector according to Claim 1, **characterized in that**, on the one hand, in the region near the injector the container adapter (200) has two more opposite windows (206) and, on the other hand, the coupling element (230) has two opposite windows (236), whereby the windows (236) in the transfer-pump position (7) of the injector are in front of the windows (206).

10. Method for making a solution (3) from a solvent (1) and an active ingredient (2) in and on a disposable injector, **characterized in that**,
- prior to making the solution (3) the solvent (1) is stored in an injector-side, first cylinder-piston unit (100), while the active ingredient (2) is contained in a second cylinder-piston unit (250) under vacuum, upstream of the cylinder-piston unit (100),
- an injection needle (242), which is sealed on the second cylinder-piston unit (250), is arranged temporarily in front of the first cylinder-piston unit (100),
- to make a connection between the interior space (110) of the cylinder (101) and the interior space (252) of the cylinder (251, 257) the second cylinder-piston unit (250) is displaced towards the first cylinder-piston unit (100) when pierced,
- the solvent (1) flows over into the interior space (252) of the second cylinder-piston unit (250) where the active ingredient (2) is dissolved in the solvent (1) to form the solution (3), and
- the solution (3) is pumped into the first cylinder-piston unit (100) with the piston (261) of the second cylinder-piston unit (250).

## Revendications

1. Instrument d'injection à usage unique avec un boîtier (10), une unité piston-cylindre (100) - à remplir au moins temporairement - disposée dans celui-ci et un adaptateur de récipient amovible (200) agencé devant celle-ci, dans lequel l'adaptateur de récipient (200) supporte une unité piston-cylindre (250) également à remplir au moins temporairement, **caractérisé en ce que**
- le piston (111) de l'unité piston-cylindre (100) est disposé de façon mobile avec commande d'aspiration et de refoulement dans un cylindre (101) fermé à l'arrière - dans l'état de livraison,
- l'adaptateur de récipient (200) est fixé à l'instrument d'injection à usage unique de façon amovible au moyen d'un élément de couplage (230), dans lequel il peut coulisser longitudinalement,
- l'adaptateur de récipient (200) comporte une zone d'adaptateur (201) et une zone de récipient (221), dans lequel les deux zones (201, 221) sont séparées par un fond intermédiaire (211) avec un trou de passage (212),
- il se trouve entre le fond intermédiaire (211) et le cylindre (101) un support d'aiguille (240) présentant une aiguille d'injection (242), appliqué de façon étanche dans la région de la face frontale antérieure (103) du cylindre (101),
- la deuxième unité piston-cylindre (250) est disposée vers l'arrière dans l'adaptateur de récipient (200), laquelle est fermée hermétiquement - dans l'état de livraison - à l'avant avec un bouchon élastique (257) et à l'arrière avec un piston (261) bloqué de façon amovible contre une introduction,
- la pointe (243) de l'aiguille d'injection (242) se termine de façon étanche dans le bouchon (257),
- l'adaptateur de récipient (200) est monté de façon coulissante dans l'élément de couplage (230) vers le support d'aiguille (240) - pour établir une liaison entre l'espace intérieur (110) du cylindre côté instrument d'injection (101) et l'espace intérieur (252) de l'autre cylindre (251, 257).

2. Instrument d'injection à usage unique selon la revendication 1, **caractérisé en ce que** le cylindre (101) de la première unité piston-cylindre (100) est - dans l'état de livraison - fermé à l'arrière de façon perméable au gaz avec une membrane de filtre stérile (119).

3. Instrument d'injection à usage unique selon la revendication 1, **caractérisé en ce que** le support d'aiguille (240) est guidé de façon longitudinalement coulissante dans la zone d'adaptateur (201).

4. Instrument d'injection à usage unique selon la revendication 1, **caractérisé en ce que** la zone d'adaptateur (201) entoure au moins localement à l'avant le cylindre côté instrument d'injection (101).

5. Instrument d'injection à usage unique selon la revendication 1, **caractérisé en ce que** la deuxième unité piston-cylindre (250) est montée de façon fixe dans l'adaptateur de récipient (200).

6. Instrument d'injection à usage unique selon la revendication 1, **caractérisé en ce que** la deuxième unité piston-cylindre (250) se compose d'un tube de verre transparent (251), fermé avec un bouchon élastique (257) et un piston (261).

7. Instrument d'injection à usage unique selon la revendication 1, **caractérisé en ce que** le piston (261) de la deuxième unité piston-cylindre (250) présente des éléments d'encliquetage élastiques ou à ressort (265), qui fixent le piston (261) de façon amovible dans une position sortie.

8. Instrument d'injection à usage unique selon la revendication 1, **caractérisé en ce que** l'adaptateur de récipient (200) présente deux fenêtres opposées l'une à l'autre (226) dans la zone centrale du tube de verre (251).

9. Instrument d'injection à usage unique selon la revendication 1, **caractérisé en ce que** d'une part l'adaptateur de récipient (200) présente deux autres fenêtres opposées l'une à l'autre (206) dans la zone proche de l'instrument d'injection et d'autre part l'élément de couplage (230) présente deux fenêtres opposées l'une à l'autre (236), dans lequel les fenêtres (236) se trouvent en face des fenêtres (206) dans la position de transvasement (7) de l'instrument d'injection.

10. Procédé de préparation d'une solution (3) composée d'un solvant (1) et d'une substance active (2) dans et sur un instrument d'injection à usage unique, **caractérisé en ce que**:
- avant la préparation de la solution (3), le solvant (1) est stocké dans une première unité piston-cylindre côté instrument d'injection (100), tandis que la substance active (2) est contenue dans une deuxième unité piston-cylindre maintenue sous vide (250), placée avant l'unité piston-cylindre (100),
- une aiguille d'injection (242) est disposée temporairement devant la première unité piston-cylindre (100) et est placée de façon étanche sur la deuxième unité piston-cylindre (250)
- on déplace la deuxième unité piston-cylindre (250) vers la première unité piston-cylindre (100) avec une percée - pour l'établissement d'une liaison entre l'espace intérieur (110) du cylindre (101) et l'espace intérieur (252) du cylindre (251, 257),
- le solvant (1) s'écoule dans l'espace intérieur (252) de la deuxième unité piston-cylindre (250) et la substance active (2) s'y dissout dans le solvant (1) en formant la solution (3), et
- on pompe la solution (3) dans la première unité piston-cylindre (100) avec le piston (261) de la deuxième unité piston-cylindre (250).
